# FASCICULE DE BREVET EUROPEEN

(11) **EP 1 001 706 B1**
(45) Date de publication et mention de la délivrance du brevet: **10.03.2004**
(21) Numéro de dépôt: 98942715.8
(22) Date de dépôt: 07.08.1998
(51) Int. Cl.: A61B 10/00

(54) **DISPOSITIF DE PINCEMENT, NOTAMMENT DU TYPE PINCE A BIOPSIE**
KLEMMVORRRICHTUNG , INSBESONDERE EINE BIOPSIEZANGE
FORCEPS, IN PARTICULAR BIOPSY FORCEPS

(30) Priorité: 07.08.1997 FR 9710156
(43) Date de publication de la demande: 24.05.2000
(73) Titulaire: EUROBIOPSY, 69430 Régnié Durette (FR)
(72) Inventeur: HUGUENY, Jean-Marie, F-69430 Regnie-Durette (FR); SABIN, Pierre, Jean-Claude, F-76230 Bois Guillaume (FR); WARNIER, Antoine, F-75020 Paris (FR)
(74) Mandataire: Schmitt, John
(86) Numéro de dépôt international: PCT/FR1998/001777
(87) Numéro de publication internationale: WO 1999/007287

(56) Documents cités:
- EP-A- 0 380 874
- EP-A- 0 573 817
- US-A- 5 562 102

## Description

La présente invention a trait à un dispositif de pincement, tel que, notamment, une pince à biopsie, c'est-à-dire une pince ayant deux mors susceptibles de s'écarter, de se rapprocher et, lors du rapprochement, de couper ou détacher un échantillon corporel qui se trouve pris entre les deux mors de la pince et qui peut ensuite être récupéré à l'extérieur de l'organisme.

L'invention s'étend cependant également à d'autres appareils utilisables en médecine ou en chirurgie, tel que, par exemple, les appareils de pinçage sans effet de coupe, par exemple pour assurer un effet de clampage, ou des écarteurs, ou encore des ciseaux à usage chirurgical, ou tout autre appareil de ce genre possédant deux éléments, dont l'un, au moins, est capable de s'écarter et de se rapprocher de l'autre.

De façon typique, les pinces à biopsie actuellement connues comportent deux mors susceptibles de s'écarter ou de se rapprocher, articulés à l'extrémité d'un tube ou d'une gaine rigide ou souple allongé, à l'intérieur de laquelle peut coulisser un câble muni, à son autre extrémité, de moyens de manoeuvre par l'opérateur. Ces dispositifs comportent des biellettes articulées destinées à assurer une amplification de mouvement entre le moyen de commande constitué par le câble et les mors proprement dits.

Ces pinces sont mécaniquement complexes et comportent au moins une dizaine de pièces. Elles sont donc coûteuses, difficiles à monter et sujettes à l'usure et aux pannes. Elles entraînent, au niveau de la liaison avec le câble, des effets de torsion induisant une fatigue du câble pouvant provoquer sa rupture. D'autres pièces, également, peuvent être soumises à des contraintes exagérées, de sorte que finalement, ces pinces présentent un grand nombre d'inconvénients.

Par ailleurs, la présence des biellettes, dont une extrémité d'articulation s'étend suivant l'axe de la pince rend mal aisé l'ajout d'un outil auxiliaire tel qu'un harpon s'étendant suivant l'axe de la pince. De plus, il est extrêmement difficile de coordonner le mouvement de déplacement du harpon avec celui de l'ouverture des mors de la pince.

La présente invention se propose de remédier à ces inconvénients et de fournir un appareil de pincement, notamment une pince à biopsie, de conception extrêmement simple, avec un nombre très limité de pièces, supprimant tout effort excessif susceptible de conduire à une usure des pièces, et permettant d'obtenir, d'une façon simple, des mouvements complexes, si on le désire, tout en gardant une très grande précision des mouvements des mors et permettant de plus la mise en oeuvre aisée d'un outil auxiliaire suivant l'axe de la pince.

L'invention a pour objet un appareil de pincement tel que défini dans la revendication 1.

Suivant des modes particuliers de réalisation :
- ledit élément transversal comporte une interruption formant ledit passage et ledit élément transversal est formé par deux demi-piges coaxiales portées par l'extrémité de la gaine ;
- ledit élément transversal comporte un perçage formant ledit passage, lequel perçage s'étend sensiblement perpendiculairement à l'axe de l'élément transversal ;
- ledit élément transversal comporte deux protubérances qui sont venues de matière avec ladite gaine et font saillie depuis la surface interne de l'extrémité de la gaine ;
- chaque protubérance délimite une rampe dont la normale est orientée vers l'extrémité de la gaine, chaque rampe étant prolongée par un épaulement pour le glissement de ladite surface interne dudit bras ;
- il comporte un outil auxiliaire passant au travers du passage de l'élément transversal ;
- ladite tête est prolongée suivant l'axe A-A de la gaine par ledit outil auxiliaire qui est solidaire de celle-ci, lequel outil est reçu mobile en translation dans ledit passage de l'élément transversal lors du déplacement de l'élément de manoeuvre dans la gaine ;
- ledit outil auxiliaire est une tige rigide effilée à son extrémité et formant un harpon ;
- la tige est vissée dans un trou taraudé de ladite tête ;
- ladite cavité creuse concave est sensiblement sphérique et ladite tête est une rotule ;
- ladite cavité creuse concave est au moins partiellement en forme de canal et ladite tête est sensiblement cylindrique ;
- lesdites mâchoires comportent extérieurement des reliefs adaptés pour la saisie d'un objet creux lorsque lesdites mâchoires sont écartées dans ledit objet creux ;
- lesdits mors sont isolés électriquement de ladite surface extérieure de la gaine et lesdits mors comportent des moyens de connexion à une source de potentiel ;
- les deux mors sont isolés électriquement l'un de l'autre et comportent des moyens de liaison à des sources de potentiels différents ; et
- ladite tête et ladite cavité creuse concave sont sphériques et forment une rotule pour l'articulation des mors.

De préférence, l'extrémité de la gaine, notamment lorsque la gaine est souple, est formée d'un élément rigide fixé sur une extrémité de la gaine proprement dite par une extrémité et dont l'autre extrémité, libre, de l'élément rigide porte l'élément transversal ou pige, de préférence au niveau de deux prolongements en forme de créneaux de façon que l'axe géométrique de la pige soit situé au voisinage du niveau des fonds des créneaux.

De préférence, ladite surface interne de l'extrémité de la gaine. ou de son élément d'extrémité rigide, est plane et les surfaces interne et externe du ou des bras étant alors des surfaces planes ou géométriquement cylindriques avec une génératrice parallèle à ladite surface interne de l'extrémité de la gaine et à la pige, la surface externe du renflement du bras étant également cylindrique.

Avantageusement, la section de l'extrémité de la gaine ou de son élément d'extrémité rigide, délimite intérieurement un rectangle, notamment un carré, ladite surface interne de l'extrémité de la gaine, ou de son élément d'extrémité rigide, étant engendrée par un côté du rectangle.

On peut ainsi, grâce à l'invention donner à la surface interne du bras de mors, un profil rectiligne, ou au contraire variable, se rapprochant progressivement de l'axe de la gaine au fur et à mesure que l'on s'enfonce dans la gaine, ce qui permet de donner au mouvement d'écartement du mors une cinématique correspondant à la forme de la rampe ainsi réalisée. La face externe dudit bras est alors conformée, en se rapprochant également de l'axe, de préférence de façon à constamment glisser sur l'extrémité de la surface interne de la gaine et éviter ainsi un débattement libre du bras entre ladite pige et la surface interne de la gaine.

Le mors présente, au-delà du bras, la partie formant la mâchoire proprement dite, qui peut être de toute forme appropriée à l'usage que l'on veut en faire.

La mâchoire de mors peut, par exemple, être réalisée sous forme d'une cuiller à pourtour coupant dans le cas d'une pince de biopsie.

En variante, la forme de la mâchoire peut être celle d'un mors plat. par exemple pour réaliser un effet de serrage ou de clampage.

En variante encore, la mâchoire peut se présenter sous forme d'une lame coupante de ciseau pour réaliser un instrument de sectionnement.

En général les deux mors sont symétriques et animés de mouvements parfaitement symétriques par rapport à un plan passant par l'axe de l'extrémité de la gaine et l'axe de la pige.

Cependant, dans une variante, l'un des mors peut être réalisé de façon à rester fixe en rotation et n'être animé que d'un mouvement de coulissement rectiligne pendant que l'autre est animé d'un mouvement de coulissement et de pivotement par rapport à la gaine.

Lors de l'utilisation, on peut souhaiter que le mors, en s'écartant, se déplace également axialement par rapport à l'élément à traiter, par exemple un organe. Dans ce cas, la gaine restera fixe par rapport à l'organe à traiter et le moyen de manoeuvre tel que le câble sera animé d'un mouvement de translation dans la gaine.

Au contraire, on peut souhaiter que les mors soient animés uniquement d'un mouvement d'écartement et de rapprochement par rapport à l'objet ou organe à traiter et, dans ce cas, c'est l'élément de manoeuvre qui restera fixe tandis que la gaine sera déplacée en translation le long de cet élément de manoeuvre allongé.

A cet effet, l'élément de manoeuvre et la gaine comportent des moyens adaptés pour respectivement jouer tour à tour le rôle de moyens de maintien en position de l'un de l'élément de manoeuvre ou de la gaine par rapport à l'organe à traiter et le rôle de moyens de déplacement de l'autre.

On comprend que l'on a ainsi réalisé un dispositif de pincement d'une très grande simplicité mécanique et qui comprend un nombre très limité de pièces, à savoir les deux mors, la pige transversale, la tête qui termine la gaine, et éventuellement un tronçon tubulaire rapporté sur l'extrémité de la gaine et formant la surface interne de guidage et recevant, à son extrémité, la pige.

Il en résulte un montage et un démontage facile, une simplicité d'entretien et une quasi suppression des risques de rupture ou de panne.

D'autres avantages et caractéristiques de l'invention apparaîtront à la lecture de la description suivante, faite à titre d'exemple non limitatif et se référant au dessin annexé dans lequel :
- la figure 1 est une vue en perspective éclatée du dispositif selon l'invention,
- la figure 2 est une vue en coupe axiale, dans un plan perpendiculaire à la pige, du dispositif,
- la figure 3 est une vue en coupe axiale du dispositif dans un plan axial de la pige,
- la figure 4 est une vue analogue à la figure 2 en position écartée des mors,
- la figure 5 est une vue en perspective éclatée d'une variante de réalisation du dispositif selon l'invention,
- la figure 6 est une vue en perspective d'une variante de réalisation de l'embout sur lequel les mors sont articulés ;
- la figure 7 est une vue analogue à celle de la figure 4 d'un autre mode de réalisation de l'invention ; et
- la figure 8 est une vue en perspective d'une variante de réalisation de l'élément tubulaire formant le corps de la pince.

La pince à biopsie représentée sur les figures comporte une gaine allongée 1 à l'intérieur de laquelle peut se déplacer longitudinalement un câble semi-rigide 2 dont l'une des extrémités, non représentée, est reliée à une poignée de manoeuvre de type usuel dans ce genre de pince Avantageusement, il est prévu des moyens de maintien en position par rapport à l'organe à traiter de l'un ou l'autre de la gaine et du câble et des moyens de déplacement de celui non maintenu de la gaine et du câble.

A son extrémité, la gaine 1 présente un élément tubulaire métallique 3, dont la surface extérieure est cylindrique, et qui comporte un passage central de section rectangulaire ou carrée 4. A son extrémité éloignée de la gaine, la pièce cylindrique 3 est découpée en forme de créneaux de façon à former deux prolongements 5 traversés par des trous alignés 6 dont l'axe géométrique commun est situé légèrement au-dessus du plan des deux bords plus bas 7 des créneaux. Ainsi, la partie inférieure des trous 6 se situe sensiblement dans le plan de ces bords les plus bas. Dans les trous 6 est chassée une pige cylindrique 8 qui s'étend ainsi transversalement à l'extrémité supérieure du passage de section carrée 4.

La pince comporte deux mors 9 identiques dont les parties formant les mâchoires proprement dites 10 s'étendent au-delà de l'extrémité crénelée de la pièce tubulaire 3 et présentent, d'une façon usuelle, des cavités 11 bordées par une lèvre coupante périphérique 12. Les mors 9 se prolongent chacun, à l'intérieur de la pièce 3, par un bras 13 qui passe entre la pige 8 et la surface interne plane du passage 4 au regard de la pige.

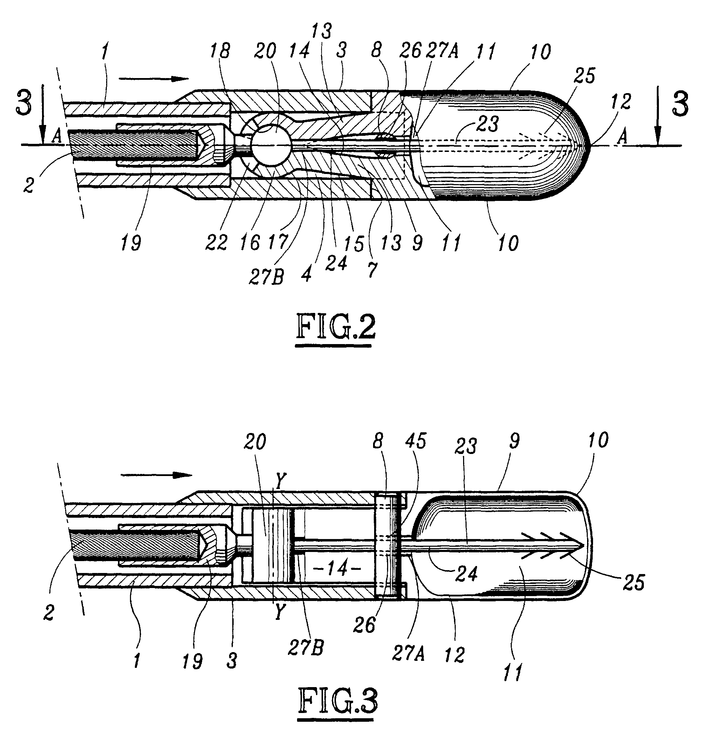

Le bras 13 présente une surface interne 14 engendrée par une génératrice perpendiculaire au plan de la figure 2 et qui, depuis la pige 8, se rapproche progressivement de l'axe A-A du dispositif jusqu'à venir pratiquement tangenter cet axe. Au-dessus de la pige, dans la position représentée sur la figure 2, la surface 14 s'incurve à nouveau en direction de l'axe et cette fois-ci vers le haut de façon à sensiblement entourer la pige 8 comme on le voit bien sur la figure 2.

Les bras 13 présentent également une surface extérieure 15, qui tend également à se rapprocher de l'axe A-A lorsque l'on descend vers le bas et qui est telle que l'épaisseur du bras 13, dans la direction perpendiculaire à l'axe A-A, comprise dans le plan de la figure 2, tend progressivement à diminuer, cette épaisseur étant presque égale à la distance située entre la pige 8 et la paroi interne plane de la pièce 3 au niveau du bord 7.

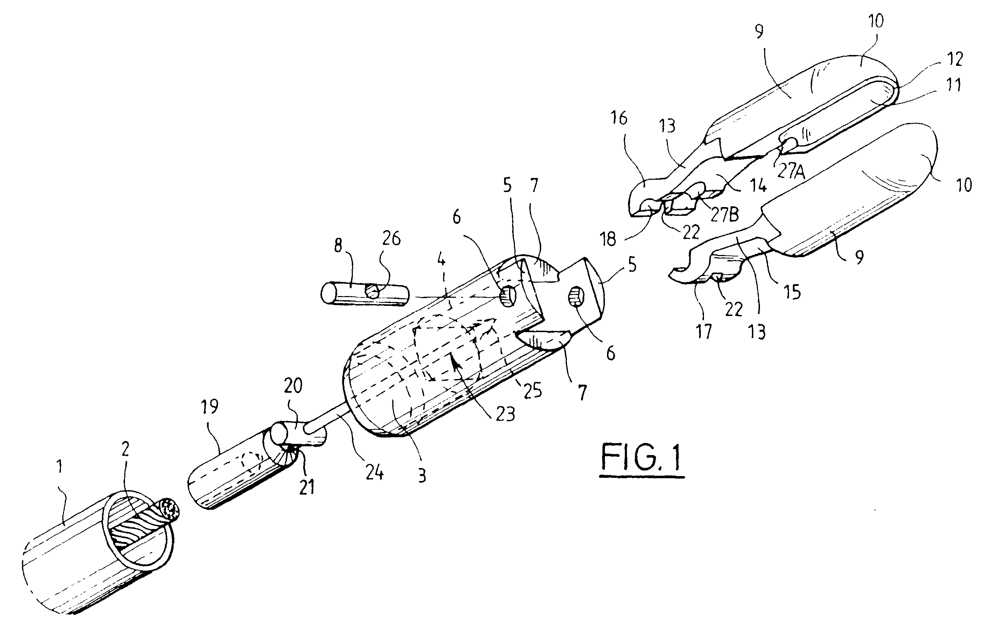

Le bras 13 présente un renflement convexe d'extrémité 16 dont la surface externe 17 a sensiblement la forme d'un cylindre à base circulaire. dont le diamètre est un peu inférieur à la distance séparant les deux surfaces internes planes, en regard, de la pièce 3. Le renflement 16 présente, à l'intérieur, une cavité creuse concave 18 non sensiblement sphérique. Cette cavité 18 est, par exemple, formée, dans le mode de réalisation des figures 1 à 4, par un canal s'étendant suivant l'axe du cylindre délimitant la surface externe 17. Ainsi, l'axe du canal délimitant la cavité 18 s'étend perpendiculairement à l'axe A-A et parallèlement à la pige 8.

L'extrémité du câble 2 porte un embout 19 fixé sur le câble et présentant, à son extrémité libre, une tête 20 qui est reçue dans la réunion des deux cavités 18 des deux mors 9.

La tête 20 est non sensiblement sphérique. Elle est sensiblement de révolution autour d'un axe Y-Y transversal perpendiculaire à l'axe A-A. Elle est par exemple formée par une tête cylindrique s'étendant perpendiculairement à l'extrémité de l'embout 19. La tête cylindrique 20 est formée à l'extrémité d'une tige 21 de plus petit diamètre que la tête 20 et qui prolonge axialement l'embout 19.

La cavité creuse concave 18 destinée à la réception de la tête cylindrique 20 est alors formée par deux canaux de section semi-cylindrique, dont la génératrice s'étend parallèlement à la pige 8. La paroi latérale de chaque canal s'étend en section sur environ 145°.

Des encoches 22 sont ménagées axialement aux extrémités des renflements 16. Ces encoches 22 s'étendent perpendiculairement aux canaux 18 et sont destinées au passage de la tige 21 lorsque les renflements 16 enserrent la tête 20.

La pince comporte de plus un outil auxiliaire axial 23 tel qu'un harpon axial s'étendant suivant l'axe A-A. Le harpon 23 est formé par une tige métallique 24, qui, à son extrémité libre effilée, comporte des ergots 25 destinés à maintenir le harpon dans les chairs après pénétration de celui-ci.

Le harpon 23 est fixé sur la tête 20 suivant l'axe de l'embout 19. Il est par exemple vissé dans un trou taraudé ménagé axialement dans l'embout 19.

En outre, la pige 8 comporte un passage 26 ménagé axialement destiné à la circulation de la partie courante du harpon 23. Comme représenté sur les figures 1 à 4, le passage 26 est formé par un perçage transversal ménagé dans l'épaisseur de la pige 8.

Les mors 9 comportent intérieurement dans leur zone de liaison aux bras 13 des canaux axiaux 27A pour la réception du harpon 23 lorsque la pince est refermée. De même, des canaux axiaux 27B sont prévus sur les bras 13 dans leurs zones de liaison aux protubérances 16.

Pour le montage, les deux mors 9 sont placés convenablement sur la tête 20 puis ils sont introduits dans la pièce 3 et on met enfin en place la pige 8.

Lorsque, partant de la position de pince fermée, représentée sur les figures 2 et 3, on repousse le câble vers le haut, dans le sens de la flèche. la tête 20 repousse les deux mors 9 vers le haut, de sorte que, rapidement. les surfaces 14, qui viennent glisser sur la pige 8, produisent un effet de rampe qui tend à écarter les deux bras 13 en les faisant pivoter autour de la tête 20, tout en maintenant les deux renflements 16 assemblés autour de la tête du fait que ces extrémités restent guidées dans le passage carré 4.

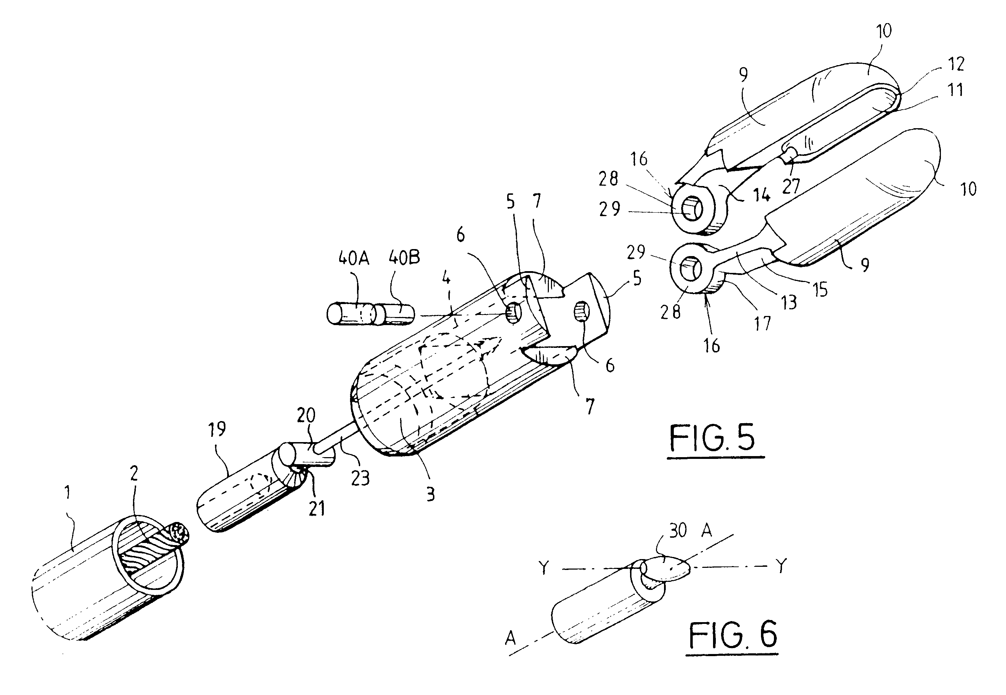

Ce mouvement de basculement des bras est progressivement autorisé du fait de l'inclinaison de la surface externe 15 des bras qui évite un coincement et guide les bras au fur et à mesure que ceux-ci montent au-dessus des rebords 7. En fin de parcours le mouvement est arrêté par la présence de la pige et les deux mors 9 se trouvent en position d'ouverture maximale représentée sur la figure 4

La fermeture des mors s'effectue en manoeuvrant le câble 2 dans le sens opposé à celui de la flèche.

La longueur du harpon 23 est adaptée afin que, lorsque les mors de la pince sont refermés, l'extrémité libre du harpon arrive sensiblement à l'extrémité avant des mors tout en permettant que le harpon soit complètement reçu à l'intérieur de la pince.

Au contraire, lorsque la pince est en position ouverte. comme représenté sur la figure 4, l'embout 19 étant avancé vers la pige 8, l'extrémité libre du harpon 23 fait saillie de quelques millimètres par rapport aux extrémités des mors. Ainsi, dans cette position, le harpon peut pénétrer dans les chairs de l'organe sur lequel la biopsie doit être pratiquée.

Après enfoncement du harpon, lorsque l'opérateur tire le câble 2 à l'intérieur de la gaine 1, le harpon solidement ancré par les ergots 25 à l'intérieur des chairs de l'organe, entraîne une partie de celui-ci entre les mors 9. Le coulissement du harpon 23 est rendu possible par la présence du passage 26. Sous l'action de la traction du câble 2, les mors 19 en se rapprochant sectionnent les chairs au-delà de l'extrémité libre du harpon. Ainsi, la partie de l'organe retenue autour du harpon est désolidarisée de l'organe et est maintenue prisonnière dans les cavités 11 des mors.

On comprend qu'on a ainsi réalisé une pince à biopsie composée d'un très petit nombre d'éléments et dont la simplicité mécanique permet de supprimer pratiquement tout risque de panne. En outre, les efforts mécaniques sur une telle pince, qui peut être très miniaturisée, sont parfaitement répartis.

De plus, dans le mode de réalisation des figures 1 à 4, après engagement de la tête cylindrique 20 entre les renflements 16, le guidage axial sans rotation de la tête 20 est assuré par le contact des surfaces cylindriques 17 sur les parois planes délimitant le passage central 4. Les formes complémentaires cylindriques de la tête 20 et des canaux 18 assurent l'articulation des mors.

En outre, la tête de pince à biopsie décrite ici présente une faible longueur axiale grâce à la simplicité de sa construction. Ainsi, elle peut circuler dans des tubes de guidage présentant de faibles rayons de courbure sans risque de coincement.

En variante, la tête de pince peut comporter des mors asymétriques. dont l'un porte par exemple une dent unique et l'autre porte deux dents délimitant un espace de réception pour la dent unique du mors complémentaire lorsque la pince est fermée.

Sur la figure 5, est représenté un autre mode de réalisation du dispositif décrit en regard des figures 1 à 4.

La variante de réalisation de la figure 5 ne diffère de celle des figures 1 à 4 qu'en ce qui concerne la pige transversale et la forme des renflements 16 et en particulier la forme des cavités creuses concaves qu'ils délimitent.

Dans ce mode de réalisation, les deux mors 9 sont identiques. Le renflement 16 de chacun d'eux est formé par un oeillet 28 à paroi latérale extérieure cylindrique. Chaque oeillet délimite intérieurement un conduit cylindrique creux 29. L'axe commun des oeillets s'étend parallèlement aux génératrices des surfaces internes 14, c'est-à-dire parallèlement à la pige 8. Chaque oeillet s'étend à l'extrémité d'un bras 13 dans le prolongement d'une face latérale correspondante. La longueur axiale (suivant l'axe de la pige 8) de chaque oeillet est sensiblement égale au tiers de la largeur du bras 13.

Dans l'appareil assemblé, les extrémités cylindriques de la tête 20 sont reçues dans les conduits cylindriques creux 29 des oeillets, ce qui assure l'articulation des mors par rapport à l'embout 19.

Suivant encore une autre variante non représentée, la protubérance 16 de chaque bras est formée sur la moitié de la largeur de chaque bras 13 par un oeillet d'extrémité prolongé par un tronçon en forme de canal analogue au canal 18 des figures 1 à 4.

Dans ce mode de réalisation, l'organe transversal 8 est formé par deux demi-piges coaxiales 40A, 40B portées chacune par un prolongement 5. Le passage est alors délimité par l'intervalle formé entre les deux demi-piges 40A, 40B.

Sur la figure 6 est représentée une variante de réalisation de l'embout 19 pouvant être mis en oeuvre dans une pince telle que décrite en regard des figures 1 à 4 d'une part et de la figure 5 d'autre part.

La tête 20, de forme sensiblement cylindrique sur les figures précédentes, est remplacée sur la figure 6 par une tête 30 non cylindrique mais de révolution autour de l'axe Y-Y qui s'étend perpendiculairement à l'axe A-A de la pince. Sur la figure 6, la tête 30 a une forme d'olive et présente deux extrémités rétrécies. La tête 30 est de révolution autour de l'axe Y-Y et est engendrée par un arc de cercle.

Avec une tête telle que représentée sur la figure 6, les cavités creuses concaves portées par les mors 9, et formées par les canaux 18 ou les conduits de révolution 29, ont des profils complémentaires à celui de la tête 30. Ainsi, les canaux 18 sont non pas engendrés par une génératrice rectiligne, mais par un arc de cercle analogue à l'arc définissant la paroi de la tête 30. De même, les passages 29 ont une section progressivement décroissante vers l'extérieur de la pince et sont engendrés par un arc de cercle de courbure analogue à l'arc de cercle engendrant la tête 30.

On comprend que la tête 30 peut prendre des profils très différents pourvu que celle-ci soit généralement de révolution autour de l'axe Y-Y s'étendant perpendiculairement à l'axe A-A de la pince. Ainsi, la courbe engendrant la tête 30 de révolution peut être quelconque.

Le profil intérieur des canaux 18 ou des conduits 29 est adapté en conséquence et présente une forme de révolution complémentaire à celle de la tête portée par l'embout 19.

Dans tous les cas, la forme allongée de la tête autour de laquelle s'articulent les mors garantit une fixité axiale de ceux-ci par rapport à la pièce 3.

En variante non représentée, la tête est sphérique et forme une rotule. Dans ce cas, les renflements 16 comportent une cavité sphérique sensiblement complémentaire pour la réception de la tête. Les.mors sont alors libres en rotation par rapport à l'axe de l'embout 19.

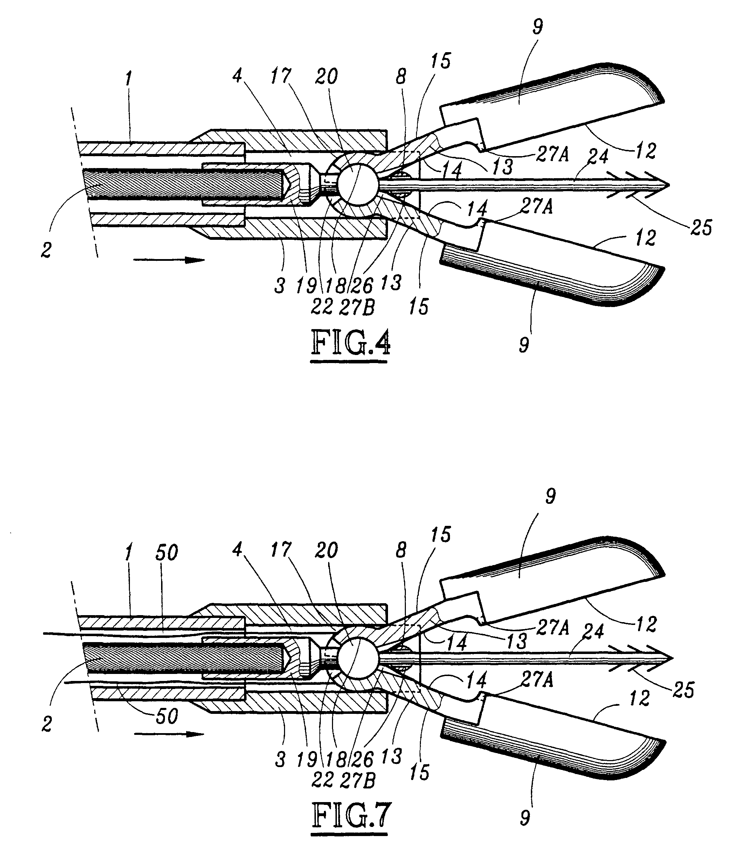

Suivant encore une variante de réalisation du dispositif représentée sur la figure 7, la gaine 1, et en particulier sa surface extérieure, sont réalisées en un matériau électriquement isolant. En outre, les deux mors 9 sont réalisés dans un matériau conducteur de l'électricité et comportent des moyens de connexion à une source de potentiel. Ces moyens de connexion sont par exemple formés par des fils conducteurs isolés 50 dont une extrémité est soudée sur les mors et dont la longueur s'étend suivant la longueur de la gaine 1. Ces fils s'étendent dans l'espace inter-paroi délimité entre la gaine 1 et le câble 2.

Lors de l'utilisation du dispositif, l'établissement d'une différence de potentiel entre d'une part les mors métalliques en contact avec les chairs du patient et d'autre part une électrode appliquée sur le corps du patient permet une coagulation des chairs dans la partie en contact avec les mors de la pince.

En variante, les deux mors sont isolés électriquement l'un de l'autre et sont reliés à des sources de potentiels différentes de sorte qu'une différence de potentiel peut être établie entre les deux mors. Un tel dispositif permet également une coagulation des chairs enserrées entre les deux mors.

Dans ce cas, l'embout 19 et la pige 8 sont réalisés en un matériau isolant tel que de la céramique.

En outre, seule une plage limitée des mors entrant en contact avec les chairs du patient peut être conductrice de l'électricité, le reste de la structure des mors étant réalisé dans un matériau isolant.

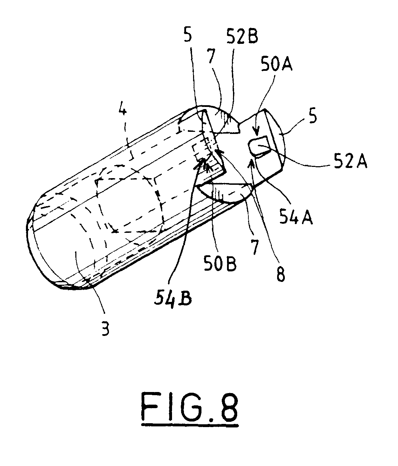

Sur la figure 8 est représentée une variante de réalisation de l'élément tubulaire désigné par la référence générale 3.

Dans ce mode de réalisation, l'organe transversal désigné par la référence générale 8 est formé de deux protubérances 50A, 50B faisant saillie dans le passage central 4 de section carrée.

Les protubérances 50A, 50B sont venues de matière avec l'élément tubulaire 3. Elles sont portées par les surfaces en regard des prolongements 5. Elles sont situées légèrement au-dessus du plan défini par les deux bords plus bas des créneaux 7.

Les protubérances 50A, 50B sont symétriques par rapport à l'axe de la pince. Elles délimitent chacune une rampe 52A, 52B, dont la normale et orientée vers l'avant de la pince. Les rampes 52A, 52B sont définies chacune latéralement par des flancs s'étendant parallèlement à l'axe de la pince.

A son extrémité arrière, dans sa région de hauteur maximale, chaque protubérance est bordée par une surface latérale semi-cylindrique notée 54A, 54B dont la génératrice s'étend perpendiculairement à l'axe de la pince. Les surfaces latérales semi-cylindriques 54A, 54B forment des épaulements définissant des surfaces de came pour les surfaces internes 14 des bras. La hauteur des surfaces 54A, 54B est sensiblement égale à 0,1 mm pour une pince dont le diamètre externe est de 2 mm.

Dans une pince à biopsie comportant l'élément tubulaire 3, les autres organes sont identiques à ceux décrits en regard, par exemple, de la figure 1.

Pour l'assemblage d'une telle pince, les bras 13 sont engagés de part et d'autre de la tête 20. La tête 20 et les renflements convexes d'extrémité 16 des bras sont introduits dans le passage 4 depuis l'extrémité avant de l'élément tubulaire 3. Lors de leur introduction, les bras 13 entrent en contact avec les rampes 52A, 52B, ce qui provoquent l'écartement des prolongements 5, par déformation élastique de ceux-ci. Ainsi, cette déformation de l'extrémité avant de l'élément tubulaire 3 permet l'introduction des renflements d'extrémité 16.

Après un enfoncement suffisant des bras 13 dans le passage 4, les protubérances 50A, 50B sont reçues entre les surfaces internes 14 des bras. Les protubérances assurent alors d'une part la retenue des bras 13 partiellement à l'intérieur du passage 4, et d'autre part l'écartement des mors lors du déplacement de l'embout 19 vers l'avant.

On conçoit qu'avec un élément tubulaire 3 tel que représenté sur la figure 8, l'écartement des mors est assuré par les protubérances venues de matière, ce qui réduit le nombre total de pièces de la pince et permet un assemblage plus rapide de celle-ci.

De plus, comme dans les modes de réalisation précédents, un passage est ménagé entre les deux protubérances, ce qui permet l'acheminement d'un outil auxiliaire entre les deux mors.

Avantageusement, l'élément tubulaire représenté sur la figure 8 peut être mis en oeuvre avec un embout 19 dont la tête est sphérique et forme une rotule. Dans ce cas, les renflements 16 comportent une cavité sphérique pour la réception de la tête.

On comprend qu'avec une pince, qui est dépourvue de biellette ou autre mécanisme complexe faisant saillie dans certaines positions au dehors de l'enveloppe généralement cylindrique de la pince, il est possible que la gaine isolante 1 soit disposée très en avant sur l'élément tubulaire 3, de sorte que la gaine recouvre complètement celui-ci et que seuls les mors conducteurs soient exposés.

Enfin, suivant encore une autre variante, les mors 9 comportent sur leur face latérale extérieure des reliefs, notamment des crochets ou des rugosités destinés à la saisie d'organes creux. Un tel dispositif est notamment destiné au retrait de débris de cathéter dans une veine ou une artère. Le dispositif fonctionne alors comme un écarteur et permet la saisie d'objets creux lorsque les mâchoires, après introduction à l'état resserré, sont écartées à l'intérieur de celui-ci et que les reliefs sont en contact avec la surface interne de l'objet creux.

## Revendications

1. Appareil de pincement, notamment du type pince à biopsie, comprenant, à l'extrémité d'une gaine (1, 3) rigide ou non, dans laquelle peut coulisser un élément allongé de manoeuvre (2), de préférence un câble. deux mors (9), dont l'un, au moins, est susceptible de s'écarter et de se rapprocher de l'autre, lorsque ledit élément de manoeuvre (2) coulisse axialement dans l'extrémité de la gaine (1, 3), le ou lesdits mors (9) présentant, dans le prolongement d'une partie de mors faisant office de mâchoires proprement dites (10), un bras (13) se rapprochant progressivement de l'axe (A-A) de la gaine (1, 3) de façon à former une première surface inclinée (14). interne. se rapprochant progressivement dudit axe (A-A) et faisant face à celui-ci, une deuxième surface inclinée (15), externe, se rapprochant également progressivement dudit axe (A-A) et faisant face à la surface interne de l'extrémité de la gaine (1, 3), ledit bras (13) présentant un renflement convexe (16) susceptible de glisser sensiblement contre ladite surface interne de l'extrémité de la gaine (1, 3), ledit renflement (16) présentant, au regard dudit axe (A-A), une cavité creuse concave (18 ; 29), ladite gaine (1. 3) présentant, vers son extrémité au-delà de laquelle s'étend ladite mâchoire proprement dite (10), un élément transversal (8), tel qu'une pige, contre lequel vient glisser ladite surface interne (14) dudit bras (13), ledit élément allongé de manoeuvre (2) se terminant par une tête (20 ; 30) logée dans ladite cavité creuse concave (18 ; 29), de sorte que lorsque ledit élément allongé de manoeuvre (2) est déplacé vers l'extrémité libre de la gaine (1, 3), il repousse ledit mors (9) au-delà de l'extrémité, amenant ainsi la surface interne (14) dudit bras (13), qui glisse sur ledit élément transversal (8), à pivoter par effet de rampe, en écartant la mâchoire du mors de l'autre mâchoire, ledit écartement étant autorisé par l'inclinaison de ladite surface externe (15) qui sort de l'extrémité de la surface interne de la gaine (1, 3), ledit renflement (16) du bras pivotant, dans ce mouvement, autour de ladite tête (20 ; 30) tout en restant guidé dans ladite gaine (1. 3). le mouvement inverse de l'élément de manoeuvre (2) provoquant le rapprochement du mors par un mouvement inverse **caractérisé en ce que** ledit élément transversal (8) comporte un passage (26) d'axe sensiblement parallèle à l'axe (A-A) de la gaine (1, 3) permettant l'acheminement d'un outil auxiliaire (23) entre les deux mors (9).

2. Appareil selon la revendication 1, **caractérisé en ce que** ledit élément transversal (8) comporte une interruption formant ledit passage et **en ce que** ledit élément transversal est formé par deux demi-piges coaxiales (40A, 40B) portées par l'extrémité de la gaine (1, 3).

3. Appareil selon la revendication 1, **caractérisé en ce que** ledit élément transversal (8) comporte un perçage (26) formant ledit passage, lequel perçage s'étend sensiblement perpendiculairement à l'axe de l'élément transversal (8).

4. Appareil selon la revendication 1, **caractérisé en ce que** ledit élément transversal (8) comporte deux protubérances (50A, 50B) qui sont venues de matière avec ladite gaine (1, 3) et font saillie depuis la surface interne de l'extrémité de la gaine (1, 3).

5. Appareil selon la revendication 4, **caractérisé en ce que** chaque protubérance (50A, 50B) délimite une rampe (52A, 52B) dont la normale est orientée vers l'extrémité de la gaine (1, 3), chaque rampe (52A, 52B) étant prolongée par un épaulement (54A, 548) pour le glissement de ladite surface interne (14) dudit bras (13) associé.

6. Appareil selon l'une quelconque des revendications précédentes, **caractérisé en ce qu'**il comporte un outil auxiliaire (23) passant au travers du passage (26) de l'élément transversal (8).

7. Appareil selon la revendication 6, **caractérisé en ce que** ladite tête (20) est prolongée suivant l'axe (A-A) de la gaine par ledit outil auxiliaire (23) qui est solidaire de celle-ci, lequel outil est reçu mobile en translation dans ledit passage (26) de l'élément transversal (8) lors du déplacement de l'élément de manoeuvre (2) dans la gaine (1, 3).

8. Appareil selon la revendication 7, **caractérisé en ce que** ledit outil auxiliaire (23) est une tige rigide (24) effilée à son extrémité et formant un harpon (23).

9. Appareil selon la revendication 8, **caractérisé en ce que** la tige (24) est vissée dans un trou taraudé de ladite tête (20).

10. Appareil selon l'une quelconque des revendications précédentes, **caractérisé en ce que** lesdites mâchoires (10) comportent extérieurement des reliefs adaptés pour la saisie d'un objet creux lorsque lesdites mâchoires sont écartées dans ledit objet creux.

11. Appareil selon l'une quelconque des revendications précédentes, **caractérisé en ce que** l'élément de manoeuvre (2) et la gaine (3) comportent des moyens adaptés pour respectivement jouer tour à tour le rôle de moyens de maintien en position de l'un de l'élément de manoeuvre (2) ou de la gaine (3) par rapport à l'organe à traiter et le rôle de moyens de déplacement de l'autre.

12. Appareil selon l'une quelconque des revendications précédentes, **caractérisé en ce que** lesdits mors sont isolés électriquement de ladite surface extérieure de la gaine (1, 3) et **en ce que** lesdits mors (9) comportent des moyens de connexion à une source de potentiel.

13. Appareil selon la revendication 12, **caractérisé en ce que** les deux mors (9) sont isolés électriquement l'un de l'autre et comportent des moyens de liaison à des sources de potentiels différents.

14. Appareil selon l'une quelconque des revendications précédentes, **caractérisé en ce que** ladite tête (20, 30) et ladite cavité creuse concave (18, 29) sont sphériques et forment une rotule pour l'articulation des mors (9).

## Claims

1. Forceps instrument, in particular of the biopsy-forceps type, comprising, on the end of an optionally rigid sheath (1, 3), in which may slide an elongate maneuvering element (2), preferably a cable, two jaw-pieces (9), one of which, at least, is capable of being moved away from and of being moved toward the other when said maneuvering element (2) slides axially in the end of the sheath (1, 3), **characterized in that** said jaw-piece or jaw-pieces (9), having, in the extension of a part of the jaw-pieces acting as the jaws proper (10), an arm (13), going progressively toward the axis (A-A) of the sheath (1, 3) so as to form a first, internal, inclined surface (14), going progressively toward said axis (A-A) and facing the latter, a second, external, inclined surface (15), also going progressively toward said axis (A-A) and facing the internal surface of the end of the sheath (1, 3), said arm (13) having a convex excrescence (16) capable of sliding substantially against said internal surface of the end of the sheath (1, 3), said excrescence (16) having, facing said axis (A-A), a concave hollow cavity (18; 29), said sheath (1, 3) having, near its end beyond which said jaw proper (10) extends, a transverse element (8), such as a rod against which said internal surface (14) of said arm (13) slides, and said elongate maneuvering element (2) terminating in a head (20; 30) housed in said concave hollow cavity (18; 29) so that, when said elongate maneuvering element (2) is moved toward the free end of the sheath (1, 3), it pushes said jaw-piece (9) back beyond the end, thus causing the internal surface (14) of said arm (13), which slides over said transverse element (8), to pivot by a ramp effect, moving the jaw of the jaw-piece away from the other jaw, said movement away being allowed by the inclination of said external surface (15) which protrudes from the end of the internal surface of the sheath (1, 3), said excrescence (16) of the arm pivoting, during this movement, about said head (20; 30) while still being guided in said sheath (1, 3), the reverse movement of the maneuvering element (2) causing the jaw-piece to move closer by a reverse movement, **characterized in that** said transverse element (8) includes a passage (26) of axis substantially parallel to the axis (A-A) of the sheath (1,3), for putting an auxiliary tool (23) between the two jaw-pieces (9).

2. Instrument according to Claim 1, **characterized in that** said transverse element (8) includes an interruption forming said passage, and **in that** said transverse element is formed by two coaxial half-rods (40A, 40B) carried by the end of the sheath (1, 3).

3. Instrument according to Claim 1, **characterized in that** said transverse element (8) includes an orifice (26) forming said passage, which orifice extends substantially perpendicular to the axis of the transverse element (8).

4. Instrument according to Claim 1, **characterized in that** said transverse element (8) has two protuberances (50A, 50B) which are integral with said sheath (1, 3) and project from the internal surface of the end of the sheath (1, 3).

5. Instrument according to Claim 4, **characterized in that** each protuberance (50A, 50B) delimits a ramp (52A, 52B) whose normal is directed toward the end of the sheath (1, 3), each ramp (52A, 52B) being extended by a shoulder (54A, 54B) for the sliding of said internal surface (14) of said associated arm (13).

6. Instrument according to any one of the preceding claims, **characterized in that** it includes an auxiliary tool (23) passing through the passage (26) of the transverse element (8).

7. Instrument according to Claim 6, **characterized in that** said head (20) is extended along the axis (A-A) of the sheath by said auxiliary tool (23), which is secured to it, which tool is accommodated such that it can move in translation in said passage (26) of the transverse element (8) when the manoeuvring element (2) is moved in the sheath (1, 3).

8. Instrument according to Claim 7, **characterized in that** said auxiliary tool (23) is a rigid shaft (24) tapered at its end and forming a barb (23).

9. Instrument according to Claim 8, **characterized in that** the shaft (24) is screwed into a threaded hole in said head (20).

10. Instrument according to any one of the preceding claims, **characterized in that** said jaws (10) have reliefs on the outside designed for gripping a hollow object when said jaws are moved apart in said hollow object.

11. Instrument according to any one of the preceding claims, **characterized in that** the manoeuvring element (2) and the sheath (3) include means which are designed to act, in turn, as means of holding one of the manoeuvring element (2) and the sheath (3) in position with respect to the organ to be treated and as means of moving the other of these, respectively.

12. Instrument according to any one of the preceding claims, **characterized in that** said jaw-pieces are electrically insulated from said outer surface of the sheath (1,3), and **in that** said jaw-pieces (9) have means for connection to a source of potential.

13. Instrument according to Claim 12, **characterized in that** the two jaw-pieces (9) are electrically insulated from one another and have means for connection to sources of different potentials.

14. Instrument according to any one of the preceding claims, **characterized in that** said head (20, 30) and said concave hollow cavity (18, 29) are spherical and form a ball-and-socket joint for the articulation of the jaw-pieces (9).

## Patentansprüche

1. Klemmvorrichtung, insbesondere der Biopsiezangenart, mit am Ende einer gegebenenfalls starren Hülle (1, 3), in der ein längliches Betätigungselement (2), vorzugsweise ein Kabel, gleiten kann, zwei Spannbacken (9), von denen mindestens eine sich von der anderen entfernen und sich ihr nähern kann, wenn das Betätigungselement (2) axial in dem Ende der Hülle (1, 3) gleitet, wobei die Spannbacke(n) (9) in der Verlängerung eines Spannbackenteils, der als die eigentlichen Klemmbacken (10) fungiert, einen Arm (13) aufweist (aufweisen), der sich der Achse (A-A) der Hülle (1, 3) allmählich nähert, um eine erste geneigte Innenfläche (14) zu bilden, die sich der Achse (A-A) allmählich nähert und zu ihr weist, wobei eine zweite geneigte Außenfläche (15) sich ebenfalls der Achse (A-A) allmählich nähert und zu der Innenfläche des Endes der Hülle (1, 3) weist, wobei der Arm (13) eine konvexe Verdickung (16) aufweist, die im Wesentlichen an der Innenfläche des Endes der Hülle (1, 3) gleiten kann, wobei die Verdickung (16) gegenüber der Achse (A-A) einen konkaven Hohlraum (18; 29) aufweist und die Hülle (1, 3) gegen ihr Ende, hinter dem sich die eigentliche Klemmbacke (10) erstreckt, ein Querelement (8), wie zum Beispiel einen Stab, aufweist, an dem die Innenfläche (14) des Arms (13) gleitet, wobei das längliche Betätigungselement (2) mit einem Kopf (20; 30) abschließt, der in dem konkaven Hohlraum (18; 29) untergebracht ist, so dass das längliche Betätigungselement (2) bei seiner Verschiebung zu dem freien Ende der Hülle (1, 3) hin die Spannbacke (9) über das Ende hinaus schiebt und so die Innenfläche (14) des Arms (13), die auf dem Querelement (8) gleitet, dazu veranlasst, durch Rampenwirkung zu schwenken, wobei die Klemmbacke der Spannbacke von der anderen Spannbacke entfernt wird, wobei das Entfernen durch die Neigung der Außenfläche (15), die aus dem Ende der Innenfläche der Hülle (1, 3) heraustritt, gestattet wird, wobei die Verdickung (16) des Arm bei dieser Bewegung um den Kopf (20; 30) schwenkt und dabei weiterhin in der Hülle (1, 3) geführt wird, wobei die umgekehrte Bewegung des Betätigungselements (2) das Annähern der Spannbacke durch eine umgekehrte Bewegung bewirkt, **dadurch gekennzeichnet, dass** das Querelement (8) einen Durchgang (26) mit im Wesentlichen parallel zur Achse (A-A) der Hülle (1, 3) verlaufender Achse aufweist, der die Zuführung eines zusätzlichen Werkzeugs (23) zwischen den beiden Spannbacken (9) gestattet.

2. Vorrichtung nach Anspruch 1, **dadurch gekennzeichnet, dass** das Querelement (8) eine Unterbrechung aufweist, die den Durchgang bildet, und dass das Querelement durch zwei koaxiale Halbstäbe (40A, 40B) gebildet wird, die durch das Ende der Hülle (1, 3) getragen werden.

3. Vorrichtung nach Anspruch 1, **dadurch gekennzeichnet, dass** das Querelement (8) mit einer Bohrung (26) versehen ist, die den Durchgang bildet, wobei sich die Bohrung im Wesentlichen senkrecht zur Achse des Querelements (8) erstreckt.

4. Vorrichtung nach Anspruch 1, **dadurch gekennzeichnet, dass** das Querelement (8) zwei Vorsprünge (50A, 50B) aufweist, die einstückig mit der Hülle (1, 3) ausgebildet sind und von der Innenfläche des Endes der Hülle (1, 3) vorragen.

5. Vorrichtung nach Anspruch 4, **dadurch gekennzeichnet, dass** jeder Vorsprung (50A, 50B) eine Rampe (52A, 52B) begrenzt, deren Normale zum Ende der Hülle (1, 3) ausgerichtet ist, wobei jede Rampe (52A, 52B) durch eine Schulter (54A, 54B) zum Gleiten der Innenfläche (14) des zugehörigen Arms (13) verlängert ist.

6. Vorrichtung nach einem der vorhergehenden Ansprüche, **dadurch gekennzeichnet, dass** sie ein zusätzliches Werkzeug (23) aufweist, das sich durch den Durchgang (26) des Querelements (8) erstreckt.

7. Vorrichtung nach Anspruch 6, **dadurch gekennzeichnet, dass** der Kopf (20) durch das zusätzliche Werkzeug (23), das mit ihm einstückig ausgebildet ist, entlang der Achse (A-A) der Hülle verlängert ist, wobei das Werkzeug bei der Verschiebung des Betätigungselements (2) in der Hülle (1, 3) translatorisch beweglich in dem Durchgang (26) des Querelements (8) aufgenommen wird.

8. Vorrichtung nach Anspruch 7, **dadurch gekennzeichnet, dass** das zusätzliche Werkzeug (23) eine starre Stange (24) ist, die an ihrem Ende konisch zuläuft und ein Hakenglied (23) bildet.

9. Vorrichtung nach Anspruch 8, **dadurch gekennzeichnet, dass** die Stange (24) in ein Gewindeloch des Kopfs (20) geschraubt ist.

10. Vorrichtung nach einem der vorhergehenden Ansprüche, **dadurch gekennzeichnet, dass** die Klemmbacken (10) außen Erhöhungen aufweisen, die zum Ergreifen eines hohlen Objektes, wenn die Spannbacken in dem hohlen Objekt gespreizt werden, ausgeführt sind.

11. Vorrichtung nach einem der vorhergehenden Ansprüche, **dadurch gekennzeichnet, dass** das Betätigungselement (2) und die Hülle (3) Mittel aufweisen, die dazu ausgeführt sind, nacheinander als Mittel zum Festhalten entweder des Betätigungselements (2) oder der Hülle (3) in Position bezüglich des zu behandelnden Organs bzw. als Mittel zur Verschiebung des jeweils anderen Glieds- der Hülle oder des Betätigungselements- zu wirken.

12. Vorrichtung nach einem der vorhergehenden Ansprüche, **dadurch gekennzeichnet, dass** die Spannbacken von der Außenfläche der Hülle (1, 3) elektrisch isoliert sind und dass die Spannbacken (9) Mittel zum Anschluss an eine Spannungsquelle aufweisen.

13. Vorrichtung nach Anspruch 12, **dadurch gekennzeichnet, dass** die beiden Spannbacken (9) elektrisch voneinander isoliert sind und Mittel zur Verbindung mit verschiedenen Spannungsquellen aufweisen.

14. Vorrichtung nach einem der vorhergehenden Ansprüche, **dadurch gekennzeichnet, dass** der Kopf (20, 30) und der konkave Hohlraum (18, 29) kugelförmig sind und ein Kugelgelenk für das Gelenk der Spannbacken (9) bilden.
